# EUROPEAN PATENT SPECIFICATION

(11) **EP 1 269 955 B1**
(45) Date of publication and mention of the grant of the patent: **25.01.2006**
(21) Application number: 02253919.1
(22) Date of filing: 06.06.2002
(51) Int. Cl.: A61F 13/496, A61F 13/15

(54) **Pants-type disposable wearing article**
Wegwerfhöschen
Couche-culotte jetable

(30) Priority: 06.06.2001 JP 2001170729
(43) Date of publication of application: 02.01.2003
(73) Proprietor: UNI-CHARM CORPORATION, Shikokuchuo-shi, Ehime-ken (JP)
(72) Inventor: Ukegawa, Kazuo, c/o Technical Center, Mitoyo-gun, Kagawa-ken 769-1602 (JP); Isono, Masato, c/o Technical Center, Mitoyo-gun, Kagawa-ken 769-1602 (JP); Kamio, Hiroaki, c/o Technical Center, Mitoyo-gun, Kagawa-ken 769-1602 (JP); Oba, Toru, c/o Technical Center, Mitoyo-gun, Kagawa-ken 769-1602 (JP)
(74) Representative: Parry, Christopher Stephen

(56) References cited:
- EP-A- 0 487 921
- EP-A- 0 641 552
- WO-A-96/27352
- US-A- 5 735 839

## Description

### BACKGROUND OF THE INVENTION

This invention relates to a pants-type disposable wearing article for absorption and containment of bodily discharges.

A conventional pants-type disposable wearing article comprises a liquid-pervious topsheet, a liquid-impervious backsheet and a liquid-absorbent core interposed between these sheets to form front and rear waist regions opposed to each other and a crotch region extending between these waist regions. A plurality of waist-surrounding elastic members are secured in a stretched state to at least the backsheet of the top- and backsheets so that these elastic members extend across the front and rear waist regions in a waist surrounding direction and are spaced one from another by a given dimension in a longitudinal direction between a peripheral edge of a waist-hole and the crotch region.

In this article of well known art, the front and rear waist regions are overlaid and connected to each other along transversely opposite side edge portions of these waist regions so as to define a waist-hole and a pair of leg-holes. In the core-free region of the article, the waist-surrounding elastic members are secured to respective inner surfaces of the top- and backsheets and, in the region of the article occupied by the core, the waist-surrounding elastic members are secured to the inner surface of the backsheet. This article of well known art utilizes tensile stress of these stretched waist-surrounding elastic members to tighten the article around the wearer's torso and to prevent the article from slipping down around the wearer's torso. Such wearing article is disclosed, for example, in Japanese Patent Application Nos. 1997-84826A and 1998-127689A.

with the above described wearing article of prior art, a plurality of gathers and wrinkles are irregularly formed as the waist surrounding elastic members contact in the front and rear waist regions of the top- and backsheets. Even if gaps are formed between the wearer's torso and the gathers generated in this manner, it can not be expected the these gaps regularly extend from the crotch region toward the waist-hole in the longitudinal direction of the article. Instead, the gaps may be interrupted and/or turned to extend in the waist-surrounding direction. In consequence, it is impossible for this article of prior art to escape stuffy moisture generated within the article outside from the waist-hole through these gaps. Furthermore, such article of prior art is disadvantageous in that the gathers and/or wrinkles are apt to be angularly bent as these gathers come in contact with the wearer's torso, so these gathers and/or wrinkles may locally compress the wearer's skin and create a feeling of uncomfortable irritation against the wearer's skin.

### SUMMARY OF THE INVENTION

It is an object of this invention to provide a pants-type disposable wearing article which is improved in that a wearer of the article can be free from any uncomfortable irritation, because stuffy moisture possibly generated within the article can be effectively escaped from inside the article.

According to this invention, there is provided a pants-type disposable wearing article comprising a chassis member forming front and rear waist regions opposed to each other and a crotch region positioned between these waist regions and a liquid-absorbent member extending across the crotch region into the front and rear waist regions, a waist-hole and a pair of leg-holes below the waist-hole and wherein a plurality of waist-surrounding elastic members extending in a waist-surrounding direction are secured in a stretched state to the chassis member in at least one of the front and rear waist regions so that the waist-surrounding elastic members are arranged to be spaced one from another at a given distance in a longitudinal direction of the article and the chassis member is provided with a plurality of gathers generated by contraction of the waist-surrounding elastic members.

The gathers are further formed at regular intervals by repeated patterns of troughs and crests of the chassis member in the waist-surrounding direction extending in the longitudinal direction from the waist-hole toward the crotch region substantially continuous and perpendicular to the waist-surrounding elastic members.

This invention includes other preferred embodiments as follows:
The chassis member presents alternately troughs and crests generated by the waist-surrounding elastic members and the maximum height of the gathers between respective trough and respective crest thereof as measured in regions each having the waist-surrounding elastic member is smaller than that as measured in regions between each pair of the adjacent waist-surrounding elastic members.
Both the crests and the troughs of the gathers are substantially in a shape of circular arcs extending in the waist-surrounding direction.
The distance between the crests of adjacent gathers in the waist-surrounding direction is in a range of 3 - 30 mm.
The maximum height of the gathers between the respective troughs and the respective crests thereof as measured in the regions each having the waist-surrounding elastic member is in a range of 1 - 3 mm while that as measured in regions between each pair of the adjacent waist-surrounding elastic members is in a range of 3 - 10 mm.
The waist-surrounding elastic members extend substantially in parallel to the peripheral edge of the waist-hole and each pair of the adjacent waist-surrounding elastic members in the longitudinal direction is spaced from each other with a distance in a range of 3 - 30 mm.
A shrinkage in the waist-surrounding direction of the sheet member in the regions having the waist-surrounding elastic members is in a range of 35 - 80 % per unit length.
The chassis member is formed by a liquid-pervious topsheet facing a wearer's skin and a liquid-impervious backsheet facing away from the wearer's skin, the liquid-absorbent member being interposed between the top- and backsheets, and the gathers extending, in a region of the chassis member not occupied by the liquid-absorbent member, practically in continuity in the longitudinal direction, i.e., substantially perpendicular to the waist-surrounding elastic members and arranged substantially at regular intervals in the waist-surrounding direction.

### BRIEF DESCRIPTION OF THE DRAWINGS

Fig. 1 is a partially cutaway perspective view showing one embodiment of a disposable wearing article according to this invention;
Fig. 2 is a partially cutaway developed plan view showing the article before assembled into the pants-type;
Fig. 3 is a sectional view taken along a line A-A in Fig. 1;
Fig. 4 is a fragmentary perspective view of a chassis member;
Fig. 5 is a cross-sectional view taken along a line B-B in Fig. 4;
Fig. 6 is a cross-sectional view taken along a line C-C in Fig. 4;
Fig. 7 is a cross-sectional view taken along a line D-D in Fig. 4;
Fig. 8 is a partially cutaway perspective view showing an alternative embodiment of the disposable wearing article according to this invention;
Fig. 9 is a cross-sectional view taken along a line E-E in Fig. 8; and
Fig. 10 is a cross-sectional view taken along a line F-F in Fig. 8.

### DETAILED DESCRIPTION OF THE PREFERRED EMBODIMENTS

Details of a pants-type disposable wearing article will be more fully understood from the description given hereunder in reference to the accompanying drawings.

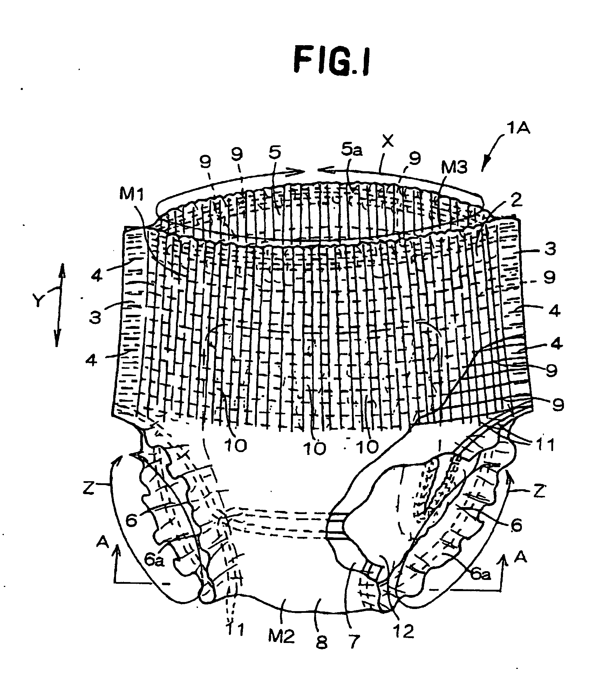

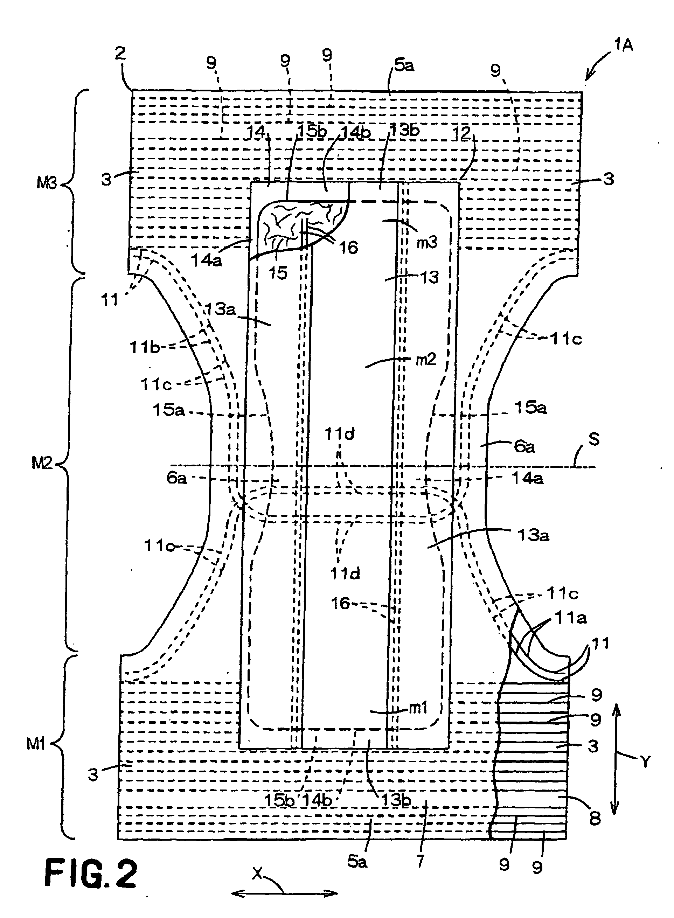

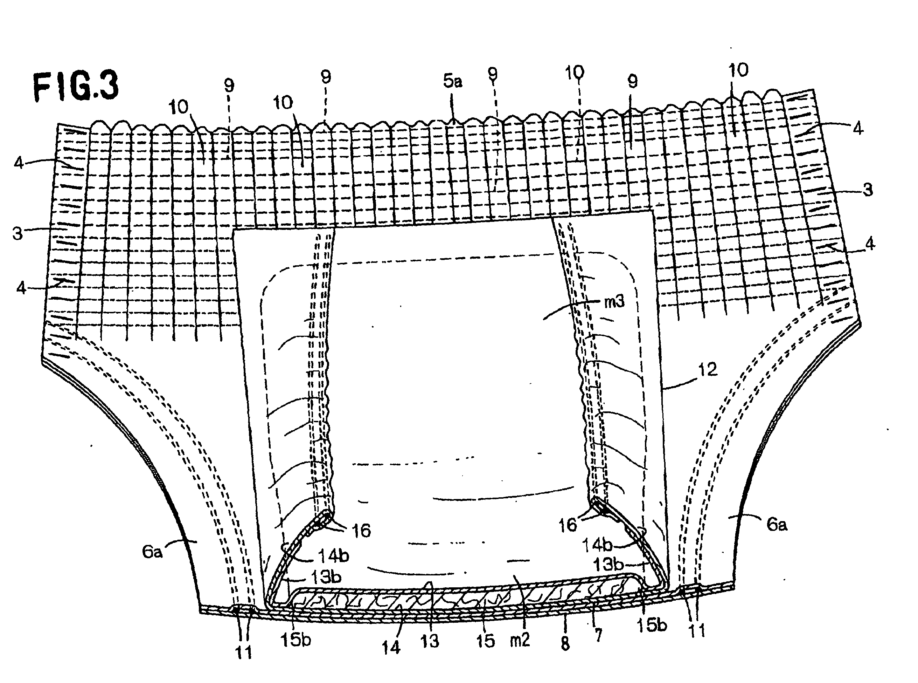

Fig. 1 is a partially cutaway perspective view showing one embodiment of a disposable wearing article 1A according to this invention, Fig. 2 is a partially cutaway developed plan view showing the article 1A before assembled into a pants-type one and Fig. 3 is a sectional view taken along a line A-A in Fig. 1. In Fig. 1, a waist-surrounding direction is indicated by an arrow X, a longitudinal direction which is substantially orthogonal to the waist-surrounding direction is indicated by an arrow Y and a thigh-surrounding direction is indicated by an arrow Z. In Fig. 2, an expression "inner surfaces" of a liquid-pervious topsheet 13 and a liquid-impervious backsheet 14 refer to the surfaces of these top- and backsheets 13, 14 facing a liquid-abeorbent core 15 and an expression "outer surfaces" of these sheets 13, 14 refer to the surfaces thereof facing away from the core 15.

The article 1A comprises a chassis member 2 and a liquid-absorbent member 12 attached to the inner side of the chassis member 2. The article 1A is composed of mutually opposed front and rear waist regions M1, M3 and a crotch region M2 extending between these two waist regions M1, M3.

Transversely opposite side edge portions 3 of the chassis member 2 are overlaid in the front and rear waist regions M1, M3 and joined together by means of a plurality of heat-sealing lines 4 arranged intermittently in the longitudinal direction along the respective side edge portions 3 and thereby a waist-hole 5 and a pair of leg-holes 6 below the waist-hole 5 are formed.

The chassis member 2 is formed by a composite nonwoven fabric comprising two layers of substantially non-stretchable and hydrophobic fibrous nonwoven fabrics 7, 8 overlaid each other, which are substantially identical in shape as well as in size. The chassis member 2 has an hourglass-like shape in its plan view. The chassis member 2 is provided in the front and rear regions M1, M3 with a plurality of waist surrounding elastic members 9 extending in the waist-surrounding direction to the longitudinal direction. These waist-surrounding elastic members 9 are attached in a stretched state to the chassis member 2.

These waist-surrounding elastic members 9 are arranged between a peripheral edge 5a of the waist-hole 5 and the crotch region M2 so as to be spaced one from another at a given distance in the longitudinal direction. The waist-surrounding elastic members 9 extend substantially in parallel to the peripheral edge 5a of the waist-hole 5. These elastic members 9 are interposed between the two layers of nonwoven fabrics 7, 8 constituting the chassis member 2 and joined to the respective layers of the nonwoven fabrics 7, 8.

Contraction of the waist-surrounding elastic members 9 generates a plurality of gathers 10 in the front and rear waist regions M1, M3 of the chassis member 2 in the longitudinal direction, which are spaced one from another at a given dimension in the waist-surrounding direction. The gathers 10 are, in other words, formed by repeated undulation of the chassis member 2 in the waist-surrounding direction due to the contraction of the waist-surrounding elastic members 9. The gathers 10 extend continuously and rectilinearly from the waist-hole 5 toward the crotch region M2 in the longitudinal direction substantially perpendicular to the waist-surrounding elastic members 9. These gathers 10 are formed substantially at regular intervals in the waist-surrounding direction.

A plurality of thigh-surrounding elastic members 11 are secured in a stretched state to peripheral edges 6a of the respective leg-holes 6. The thigh-surrounding elastic members 11 comprise elastic members 11a extending from the front waist region M1 toward the crotch region M2 in a shape of generally circular arcs and elastic members 11b extending from the rear waist region M3 toward the crotch region M2 in a shape of generally circular arcs.

These elastic members 11a, 11b include lateral portions 11c extending in the thigh-surrounding direction along the peripheral edges 6a of the respective leg-holes 6 and middle portions 11d extending across the crotch region M2. The middle portions 11d of these elastic members 11a, 11b intersect each other in the crotch region M2. These elastic members 11a, 11b are interposed between and secured to the nonwoven fabric layers 7, 8 constituting the chassis member 2.

The liquid-absorbent member 12 comprises the liquid-pervious topsheet 13 facing the wearer's skin, the liquid-impervious backsheet 14 facing away from the wearer's skin and the liquid-absorbent core 15 interposed between these sheets 13, 14 and joined to the inner surface of at least one of these sheets 13, 14.

The liquid-absorbent member 12 extends across the crotch region M2 into the front and rear waist regions M1, M3. The liquid-absorbent member 12 has a front zone m1 lying in the front waist region M1, a rear zone m3 lying in the rear waist region M3 and an intermediate zone m2 lying in the crotch region M2. Of the liquid-absorbent member 12, the front and rear zones m1, m3 are partially joined to the front and rear waist regions M1, M3 of the sheet member 12 by means of hot melt adhesive (not shown) and the intermediate zone m2 is joined to the crotch region M2 of the sheet member 12 by means of hot melt adhesive (not shown).

The top- and backsheets 13, 14 of the liquid-absorbent member 12 extend outwardly beyond transversely opposite side edges 15a of the core 15 to define respective lateral portions 13a, 14a. Along these lateral portions 13a, 14a, the top- and backsheets 13, 14 are overlaid and respective inner surfaces of these sheets 13, 14 are joined to each other. A plurality of elastic members 16 extending in the thigh-surrounding direction are attached in a stretched state to the respective lateral portions 13a, 14b. More specifically, the elastic members 16 are secured to the backsheet 14 so as to be wrapped by parts of the backsheet 14. In the front and rear zones m1, m3 of the liquid-absorbent member 12, the respective lateral portions 13a, 14a of the top- and backsheets 13, 14 are folded onto and joined to the outer surface of the topsheet 13. Contraction of the elastic members 16 causes the lateral portions 13a, 14a of the top- and backsheets 13, 14 in the intermediate zone m2 of the liquid-absorbent member 12 to uprise on the topsheet 13 and thereby to form barriers against bodily discharges.

The top- and backsheets 13, 14 of the liquid-absorbent member 12 extend outwardly beyond longitudinally opposite ends 15b of the core 15 to define end portions 13b, 14b. Along these end portions 13b, 14b, the top- and backsheets 13, 14 are overlaid with respective inner surfaces thereof joined to each other.

To obtain the article 1A of Fig. 1 from an article shown in Fig. 2 as a developed plan view, the article may be folded along a transverse center line S extending across the crotch region M2 to overlay the front and rear waist regions M1, M3 upon each other with the liquid-absorbent member 12 inside and then to join these waist regions M1, M3 together along the side edge portions 3 of the chassis member 2 in the front and rear waist regions M1, M3.

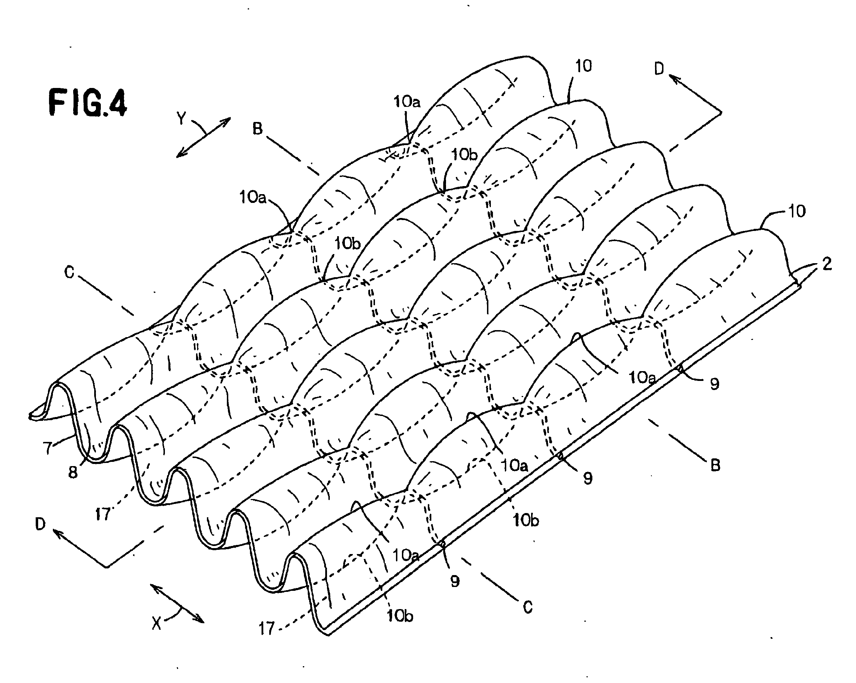

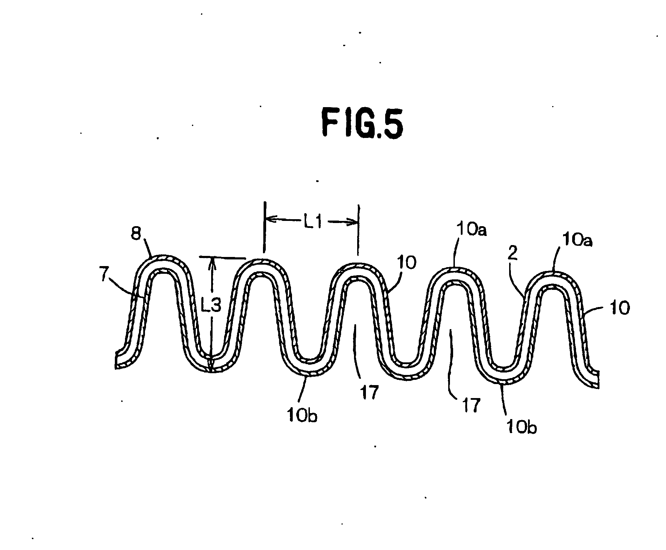

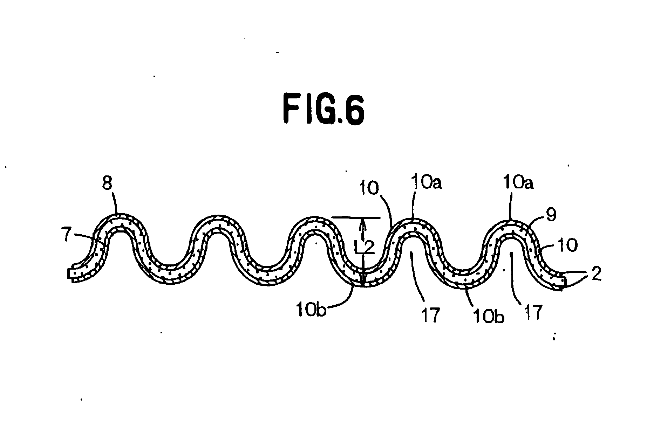

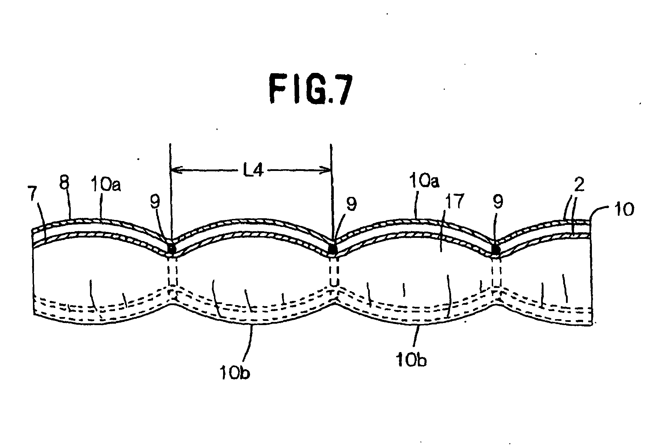

Fig. 4 is a fragmentary perspective view of the chassis member cut off from the article 1A, Fig. 5 is a cross-sectional view taken along a line B-B in Fig. 4, Fig. 6 is a cross-sectional view taken along a line C-C in Fig. 4 and Fig. 7 is a cross-sectional view taken along a line D-D in Fig. 4. In Fig. 4, the waist-surrounding direction is indicated by an arrow X and the longitudinal direction is indicated by an arrow Y.

Each of the gathers 10 has a crest 10a positioned on the side not touching the wearer's skin and a trough 10b positioned on the side touching the wearer's skin. Both the crest 10a and the trough 10b of each of the gathers extend in a substantially straight line in the longitudinal direction and in a shape of generally circular arcs in the waist-surrounding direction. The waist-surrounding elastic members 9 take alternatively patterns of crests and troughs in the waist-surrounding direction, thus making the chassis member 2 undulate repeatedly in the waist-surrounding direction.

When the article 1A is worn, the troughs 10b of the gathers 10 formed in the chassis member 2 come in contact with the wearer's torso and the crests 10a of the gathers 10 are spaced from the wearer's torso. Gaps 17 formed between the crests 10a and the wearer's torso continuously formed in the longitudinal direction from the crotch region M2 toward the waist-hole 5 in generally straight lines. Stuffy moisture generated within the article 1A can escape outside from the waist-hole 5 through these gaps 17.

As have already been discussed, the gathers 10 continuously extend in the longitudinal direction from the crotch region M2 toward the waist-hole 5, in generally straight lines and are spaced in the waist-surrounding direction substantially at regular intervals. Such unique arrangement of the gathers 10 is effective to prevent the gathers 10 from being angularly bent when the gathers 10 are pressed against the wearer's torso. In the article 1A, these gathers 10 serve like a cushion and there is no anxiety that these gathers 10 might locally compress the wearer's skin.

Both the crest 10a and the trough 10b of each of the gathers 10 take a shape of generally circular arcs in the waist-surrounding direction, so it is less anxious that the gathers 10, particularly the troughs 10b thereof might create a feeling of uncomfortable irritation against the wearer's skin when these troughs 10b come in contact with the wearer's skin than in the case of the gathers 10 having rather pointed crests 10a and troughs 10b.

The maximum height L2 of the gathers 10 measured from its crest 10a to its trough 10b as measured along the corresponding one of the waist-surrounding elastic members 9 is less than the maximum height L3 from its crest 10a to its trough 10b as measured between each pair of the adjacent waist-surrounding elastic members 9. In other words, both the crest 10a and the trough 10b of each of the gathers 10 slightly undulate in the longitudinal direction. Consequently, the troughs 10b of the gathers 10 having the associated waist-surrounding elastic members 9 are spaced away from the wearer's waist and only the troughs 10b or the gathers 10 positioned between pates of the adjacent waist-surrounding elastic members 9 will come in contact with the wearer's torso when the article 1A is worn. In this way, an area over which the article 1A comes in contact with the wearer's skin can be effectively reduced.

A distance L1 between adjacent crests 10a of the gathers 10 in the waist-surrounding direction is preferably in a range of 3 - 30 mm. If the distance L1 is less than 3 mm, the number of the gathers 10 formed in the waist-surrounding direction would be more than necessary and the number of the gathers 10 per unit length in the waist-surrounding direction would be correspondingly increased. As a result, stiffness of the chassis member 2 in the longitudinal direction in the front and rear waist regions M1, M3 would be excessively enhanced and flexibility of the chassis member 2 would be reduced. If the distance L1 exceeds 30 mm, on the contrary, the number and the height of the gathers 10 would be decreased and, in consequence, no sufficient gaps 17 would be formed between the crests 10a of the gathers 10 and the wearer's torso.

The maximum height L2 from the trough 10b to the crest 10a of each of the gathers 10 on a line having the waist-surrounding elastic members 9 is preferably in a range of 1 - 3 mm and the maximum height L3 from the trough 10b to the crest 10a of each of the gathers 10 defined in a region between each pair of the adjacent waist-surrounding elastic members 9 is preferably in a range of 3 - 10 mm. If the distance L2 is less than 1 mm, it is a likelihood that none of the gaps 17 might be formed between the crests 10a of the gathers 10 and the wearer's torso. If the distance L3 exceeds 10 mm, the gathers 10 would be bulky and create a feeling of discomfort against the wearer when the article 1A is worn.

A distance L4 between adjacent waist-surrounding elastic members 9 in the longitudinal direction is preferably in a range of 3 - 30 mm. If the distance L4 is less than 3 mm, too many small wrinkles would be formed on the chassis member 2 and it would be impossible to make the gathers 10 with substantially straight lines extending in the longitudinal direction on the chassis member 2. If the distance L4 exceeds 30 mm, on the contrary, it would be also impossible to make the gathers 10 fully extending across each pair of the adjacent elastic member 9 on the chassis member 2.

A shrinkage rate of the chassis member 2 in the waist-surrounding direction in such regions where the waist-surrounding elastic members 9 are attached in preferably in a range of 35 - 80 %. If the shrinkage rate is more than 80 %, irregularly shaped wrinkles would be formed on the chassis member 2 and it would be impossible to make the desired gathers 10 continuously and rather rectilinearly extending in the longitudinal direction on the chassis member 2. If the shrinkage rate is less than 35 %, on the contrary, it is a likelihood that an even gather-like configuration could not be formed on the chassis member 2.

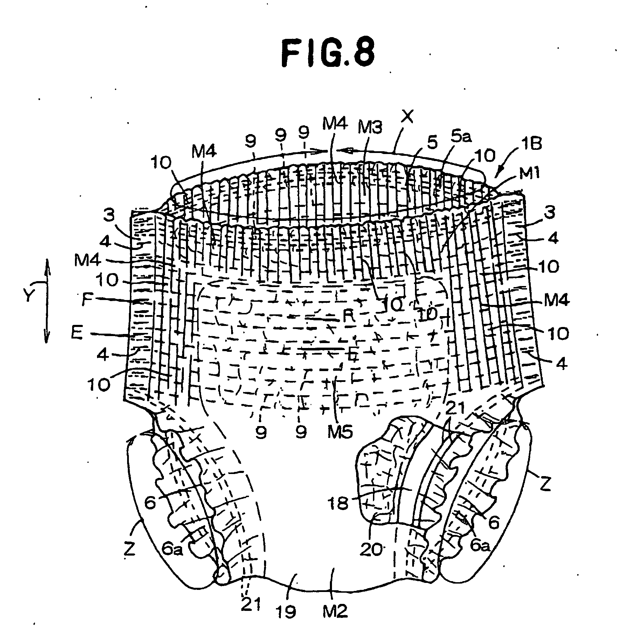

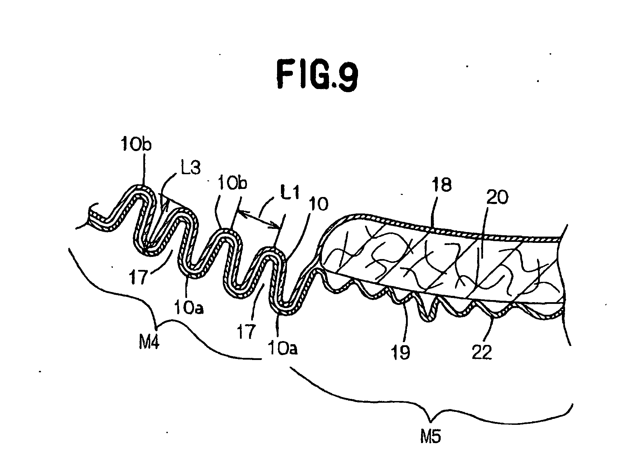

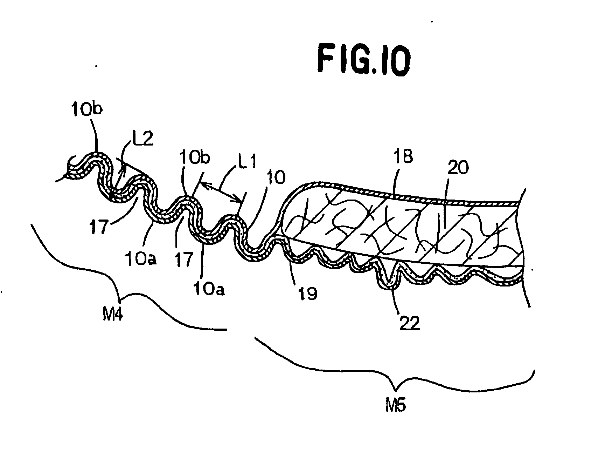

Fig. 8 is a partially cutaway perspective view showing alternative embodiment 1B of the disposable wearing article according to this invention, Fig. 9 is a cross-sectional view taken along a line E-E in Fig. 8 and Fig. 10 is a cross-sectional view taken along a line F-F in Fig. 8. In Fig. 8, the waist-surrounding direction is indicated by an arrow X, the longitudinal direction is indicated by an arrow Y and the thigh-surrounding direction is indicated by an arrow Z.

This article 1B comprises a liquid-pervious topsheet 18 facing the wearer's skin, a liquid-impervious backsheet 19 facing away from the wearer's skin and a liquid-absorbent core 20 (liquid-absorbent member) interposed between these sheets 18, 19. The core 20 is joined to the inner surface of at least one of these sheets 18, 19.

The article 1B is composed of mutually opposed front and rear waist regions M1, M3 and the crotch region M2 extending between these waist regions M1, M3. The transversely opposite side edge portions 3 of the top- and backsheets 18, 19 in the front and rear waist regions M1, M3 of the article 1B are overlaid and joined together by means of a plurality of welding lines 4 arranged intermittently in the longitudinal direction along the respective side edge portions 3 and thereby the waist-hole 5 and the pair of leg-holes 6 below the waist-hole 5 are formed. The core 20 extends across the crotch region M2 into the front and rear waist regions M1, M3. A plurality of the waist-surrounding elastic members 9 extending in the waist-surrounding direction are secured in a stretched state to the front and rear waist regions M1, M3 of the article 1B.

These waist-surrounding elastic members 9 are arranged between the peripheral edge 5a of the waist-hole 5 and the crotch region M2 so as to be spaced one from another at a given dimension in the longitudinal direction. The waist-surrounding elastic members 9 extend substantially in parallel to the peripheral edge 5a of the waist-hole 5. These elastic members 9 are interposed between the topsheet 18 and the backsheet 19. In a sub-region M4 of the front and rear waist regions M1, M3 which is free from the core 20, the waist-surrounding elastic members 9 are secured to respective inner surfaces of the top- and backsheets 18, 19. In a region M5 of the article 1B where the core 20 is present, the waist-surrounding elastic members 9 are secured to the inner surface of the backsheet 19.

A plurality of thigh-surrounding elastic members 21 extending in the thigh-surrounding direction are secured in a stretched state to peripheral edges 6a of the respective leg-holes 6. These thigh-surrounding elastic members 21 are interposed between the top- and backsheets 18, 19 and secured to the respective inner surfaces of these sheets 18, 19.

In the sub-region M4 of the front and rear waist regions M1, M3 contraction of the thigh-surrounding elastic members 9 causes the top- and backsheets 18, 19 to generate a plurality of gathers 10 spaced one from another in the waist-surrounding direction. Each of these gathers 10 is formed by alternate undulation of the top- and backsheets 18, 19. In the sub-region M4, the gathers 10 continuously and rather rectilinearly extend in the longitudinal direction from the waist-hole 5 toward the crotch region M2 so as to be perpendicular to the waist-surrounding elastic members 9. The gathers 10 are formed substantially at regular intervals in the waist-surrounding direction. The crests 10a and the troughs 10b of these gathers 10 extend in a shape of generally circular arcs in the waist-surrounding direction. In the region M5 where the core 20 is present, the backsheet 19 is formed with a plurality of irregularly shaped gathers 22.

In the sub-region M4 of the article 1B, the gaps 17 formed between the crests 10a of the gathers 10 and the wearer's torso extend from the crotch region M2 toward the waist-hole 5 in the longitudinal direction in generally straight lines. Through these gaps 17, stuffy moisture possibly generated within the article 1B can be escaped outside through the waist-hole 5.

In the sub-region M4 of the article 1B, similarly to the case shown in Fig. 1, the top- and backsheets 18, 19 present alternately troughs and crests generated by the waist-surrounding elastic members 9 in the waist-surrounding direction. In this sub-region M4 , the maximum height L2 of each of the gathers 10 from its trough 10b to its crest 10a as measured on the line having the waist-surrounding elastic members 9 is smaller than the maximum height L3 of each of the gathers 10 from its trough 10b to its crest 10a as measured between a pair of the adjacent waist-surrounding elastic members 9. In the sub-region M4, the crests 10a and the troughs 10b of the gathers 10 slightly undulate in the longitudinal direction.

In the sub-region M4 of the article 1B, the crests 10a of the gathers 10 having the associated waist-surrounding elastic members 9 are spaced away from the wearer's torso and only the troughs 10b of the gathers 10b positioned between pairs of the adjacent waist-surrounding elastic members 9 will come in contact with the wearer's torso when the article 1B is worn. In this way, an area over which the article 1B comes in contact with the wearer's skin can be effectively reduced.

The preferred ranges of the respective dimensions for the article 1B such as the distance L1 between each pair of the adjacent gathers 10 in the sub-region M4, the maximum heights L2, L3, the distance L4 between each pair of the adjacent waist-surrounding elastic members 9 and the shrinkage of the top- and backsheets 18, 19 in the waist-surrounding direction in the regions each having the waist-surrounding elastic members 9 are similar to those for the article 1A shown in Fig. 1.

For these articles 1A, 1B illustrated as preferred embodiments, the waist-surrounding elastic members 9 may be provided at least with one of the front waist region M1 and the rear waist region M3. These waist-surrounding elastic members 9 may be secured in a stretched state additionally to the crotch region M2. It is not essential for the waist-surrounding elastic members 9 in these articles 1A, 1B to extend in parallel to the peripheral edge 5a of the waist-hole 5.

The constituent nonwoven fabric layers 7, 8 of the chassis member 2 or the top- and backsheets 13, 14 may be joined together merely by means of the waist-surrounding elastic members 9. If it is desired to complement the joining effect achieved in this manner, the nonwoven fabric layers 7, 8 or the top- and backsheets 13, 14 may be partially joined together.

The waist-surrounding elastic members 9 are preferably secured to the constituent nonwoven fabric layers 7, 8 or to the top- and backsheets 13, 14 using hot melt adhesive. When the waist-surrounding elastic members 9 are secured to the nonwoven fabric layers 7, 8 or to the sheets 13, 14 using the hot melt adhesive, a coating pattern of the adhesive may be appropriately selected from various patterns inclusive of a spiral pattern or a spray pattern. In order to ensure that the waist-surrounding elastic members 9 are reliably joined to the nonwoven fabric layers 7, 8 or to the sheets 13, 14, a percentage of coated area with an adhesive is preferably in a range from 20 % to 80 % of the total surface area of the waist-surrounding elastic members 9. If the percentage of the adhesive coated area is less than 20%, it is a likelihood that the waist-surrounding elastic members 9 might fall off from the nonwoven fabric layers 7, 8 or the sheets 13, 14. If the percentage of the adhesive coated area exceeds 80%, on the contrary, contraction of the waist-surrounding elastic members 9 would be obstructed by the presence of the adhesive.

The topsheet 13, 18 may be formed of materials selected from a group of materials including hydrophilic or hydrophobic fibrous nonwoven fabric and plastic film having a plurality of fine pores. The backsheet 14, 19 may be formed of materials selected from a group of materials including a hydrophobic fibrous nonwoven fabric, a breathable but liquid-impervious plastic film, a composite nonwoven fabric consisting of two or more layers of hydrophobic fibrous nonwoven fabric laminated one on another and a composite sheet consisting of hydrophobic fibrous nonwoven fabric and breathable but liquid-impervious plastic film laminated with each other two-layered hydrophobic fibrous nonwoven fabric laminated with each other. It is also possible to form the backsheet 14, 19 using a composite nonwoven fabric comprising a melt blown fibrous nonwoven fabric having a high water-resistance and two layers of spun bond fibrous nonwoven fabric having high strength and flexibility with the melt blown fibrous nonwoven fabric sandwiched therebetween.

Nonwoven fabric may be selected from a group of materials including products obtained by spun lacing, needle punching, melt blowing, thermal bonding, spun bonding, chemical bonding and air-through processes. Component fibers of the nonwoven fabric may be selected from a group of materials including polyolefine-, polyester- and polyamide-based fibers and core-sheath-type and side-by-side-type conjugated fibers of polyethylene/polypropylene and polyethylene/polyester.

The chassis member 2 in the article 1A of Fig. 1 may be formed by a composite nonwoven fabric consisting of two or more layers of hydrophobic fibrous nonwoven fabric laminated one on another, a composite sheet consisting of two or more layers of breathable but liquid-impervious plastic film stretchable laminated one on another and a composite sheet consisting of hydrophobic fibrous nonwoven fabric and breathable but liquid-impervious plastic film laminated with each other.

The core 15, 20 is a mixture of fluff pulp and super-absorbent polymer particles or a mixture of fluff pulp, super-absorbent polymer particles and thermoplastic synthetic fibrous resin compressed to a desired thickness. The core 15, 20 are preferably entirely covered with and joined to tissue paper in order to prevent the core 15, 20 from losing its shape and/or to prevent polymer particles from falling off. The polymer particles may be selected from a group of materials consisting of starch-based, cellulose-based and synthetic polymer.

Joining processes for the constituent nonwoven fabric 7, 8 of the chassis member 2, the top- and backsheets 13, 14, 18, 19 and the core 15, 20 may be carried out using adhesion by means of hot melt adhesive or welding technique such as heat-sealing or sonic sealing.

The pants-type disposable wearing article according to this invention is primarily characterized by that a plurality of gaps are kept between the crests of the gathers formed on the chassis member and the wearer's torso and these gaps continuously and rather rectilinearly extend from the crotch region toward the waist-hole. These gaps allow stuffy moisture possibly generated within the article to escape outside from the waist-hole. Furthermore, the gathers contacting the wearer's torso are not apt to be angularly bent and serve as a cushion. It is not a likelihood therefore that these gathers might locally compress the wearer's skin.

With the embodiment in which the maximum height of the gather from its trough to its crest as measured in the region having the waist surrounding elastic member is dimensioned to be smaller than the maximum height in the regions as measured between each pair of the adjacent waist-surrounding elastic members, the troughs of the gathers in the regions having the respective waist-surrounding elastic members are spaced from the wearer's torso and the troughs between respective pairs of the adjacent waist-surrounding elastic members come in contact with the wearer's torso. In this way, the area over which the article comes in contact with the wearer's skin can be effectively reduced.

with the embodiment in which both the crests and the troughs of the respective gathers extend in the waist-surrounding direction in a shape of substantially circular arcs, there is no anxiety that the gathers, particularly the troughs thereof might create a feeling of uncomfortable irritation against the wearer even these troughs come in contact with the wearer's torso as has been the case with a conventional article having somewhat pointed crests and/or troughs of gathers.

## Claims

1. A pants-type disposable wearing article comprising a chassis member (2) forming front and rear waist regions (M1, M3) opposed to each other and a crotch region (M2) positioned between these waist regions and a liquid-absorbent member (12) extending across said crotch region (M2) into said front and rear waist (M1, M3) regions, a waist hole (5) and a pair of leg-holes (6) below said waist-hole (5) and wherein a plurality of waist-surrounding elastic members (9) extending in a waist-surrounding direction (X) are secured in a stretched state to said chassis member (2) in at least one of said front and rear waist regions (M1, M3) and said chassis member (2) is provided with a plurality of gathers (10) generated by contraction of said waist-surrounding elastic members (9), **characterized in that**
said waist-surrounding elastic members (9) are arranged between a peripheral edge (5a) of said waist-hole (5) and the crotch region (M2) and are arranged to be spaced one from another at a given distance in a longitudinal direction of said article substantially in parallel to said peripheral edge (5a) said gathers (10) being formed at regular intervals by repeated patterns of troughs (10b) and crests (10a) of said chassis member (2) in said waist-surrounding direction (X) extending in said longitudinal direction (Y) from said waist-hole (5) to said crotch region (M2) and perpendicularly to said waist-surrounding elastic members (9), wherein gaps (17) formed between crests (10a) and a wearer's torso are continuously formed in the longitudinal direction (Y) from the crotch region (M2) to the waist-hole (5) in generally straight lines, and wherein a shrinkage in the waist-surrounding direction (X) of said chassis member (2) in said regions (M1, M3) having said waist-surrounding elastic members (9) is in a range of 35-80% per unit length.

2. The article according to Claim 1, wherein said chassis member (2) presents alternately troughs (10b) and crests (10a) generated by said waist-surrounding elastic members (9) and the maximum height (L2) of said gathers (10) between respective troughs (10b) and respective crests (10a) thereof as measured in regions each having said waist-surrounding elastic member (9) is smaller than that (L3) as measured in regions between each pair of the adjacent waist-surrounding elastic members (9).

3. The article according to Claim 1, wherein both the crests (10a) and the troughs (10b) of said gathers (10) are substantially in a shape of circular arcs extending in said waist-surrounding direction (X).

4. The article according to Claim 1, wherein the distance between the crests (10a) of said adjacent gathers (10) in said waist-surrounding direction (X) is in a range of 3-30mm.

5. The article according to Claim 2, wherein said maximum height (L2) of said gathers (10) between the respective troughs (10b) and the respective crests (10a) thereof as measured in said regions each having said waist-surrounding elastic member (9) is in a range of 1-3mm while that (L3) as measured in regions between each pair of the adjacent waist-surrounding elastic members (9) is in a range of 3-10mm.

6. The article according to Claim 1, wherein said waist-surrounding elastic members (9) extend substantially in parallel to said peripheral edge (5a) of said waist-hole (5) and each pair of said adjacent waist-surrounding elastic members (9) in said longitudinal direction is spaced from each other with a distance in a range of 3-30mm.

7. The article according to Claim 1, wherein said chassis member is formed of a liquid-pervious topsheet (18) facing a wearer's skin and a liquid-impervious backsheet (19) facing away from the wearer's skin, said liquid-absorbent member (20) being interposed between said top- (18) and backsheets (19), and wherein said gathers (10) extend, in a region of said chassis member not occupied by said liquid-absorbent member (20), practically in continuity in said longitudinal direction (Y), i.e., substantially perpendicular to said waist-surrounding elastic members (9) and arranged substantially at regular intervals in said waist-surrounding direction (X).

## Patentansprüche

1. Höschen-artiger Wegwerfkleidungs-Artikel mit einem Grundelement (2), das einander gegenüberliegend einen vorderen und einen hinteren Taillenbereich (M1, M3) sowie einen zwischen diesen Taillenbereichen angeordneten Schrittbereich (M2) bildet, und mit einem über den Schrittbereich (M2) in den vorderen und den hinteren Taillenbereich (M1, M3) hinein verlaufenden flüssigkeitsabsorbierenden Element (12) sowie einer Taillenöffnung (5) und einem Paar Beinöffnungen (6) unterhalb der Taillenöffnung (5), wobei im vorderen und/oder hinteren Taillenbereich (M1, M3) am Grundelement (2) mehrere Taillen-umgebende elastische Elemente (9) in gestrecktem Zustand befestigt sind, die in um die Taille umlaufender Richtung (X) verlaufen, und wobei das Grundelement (2) mit mehreren durch Kontraktion der Taillen-umgebenden elastischen Elemente (9) erzeugten Falten (10) versehen ist, **dadurch gekennzeichnet, daß**
die Taillen-umgebenden elastischen Elemente (9) zwischen einer Umfangskante (5a) der Taillenöffnung (5) und dem Schrittbereich (M2) und mit einem vorgegebenen Abstand in Längsrichtung des Artikels voneinander im wesentlichen parallel zur Umfangskante (5a) angeordnet sind, die Falten (10) in regelmäßigen Abständen durch in um die Taille umlaufender Richtung (X) wiederholte Muster von Tälern (10b) und Bergen (10a) des Grundelements (2) sowie in Längsrichtung (Y) von der Taillenöffnung (5) zum Schrittbereich (M2) und senkrecht zu den Taillen-umgebenden elastischen Elementen (9) verlaufend ausgebildet sind, wobei zwischen den Bergen (10a) und dem Körper eines Trägers befindliche Lücken (17) in im wesentlichen geraden Linien fortlaufend in Längsrichtung (Y) vom Schrittbereich (M2) zur Taillenöffnung (5) ausgebildet sind und wobei in den genannten Bereichen (M1, M3), die die Taillen-umgebenden elastischen Elemente (9) aufweisen, ein Schrumpfen in um die Taille umlaufender Richtung (X) des Grundelements (2) bezogen auf eine Einheitslänge im Bereich von 35 bis 80 % liegt.

2. Artikel nach Anspruch 1, wobei das Grundelement (2) abwechselnd Täler (10b) und Berge (10a) aufweist, die von den Taillen-umgebenden elastischen Elementen (9) erzeugt werden, und die maximale Höhe (L2) der Falten (10) zwischen ihren entsprechenden Tälern (10b) und entsprechenden Bergen (10a) in Bereichen gemessen, die jeweils ein Taillen-umgebendes elastisches Element (9) aufweisen, kleiner als die (L3) in Bereichen zwischen einem jeweiligen Paar benachbarter Taillen-umgebender elastischer Elemente (9) gemessene ist.

3. Artikel nach Anspruch 1, wobei sowohl die Berge (10a) als auch die Täler (10b) der Falten (10) im wesentlichen die Form von Kreisbögen aufweisen, die in die um die Taille umlaufende Richtung (x) verlaufen.

4. Artikel nach Anspruch 1, wobei der Abstand zwischen den Bergen (10a) der benachbarten Falten (10) in um die Taille umlaufender Richtung (X) im Bereich von 3 bis 30 mm liegt.

5. Artikel nach Anspruch 2, wobei die maximale Höhe (L2) der Falten (10) zwischen ihren entsprechenden Tälern (10b) und entsprechenden Bergen (10a) in Bereichen gemessen, die jeweils ein Taillen-umgebendes elastisches Element (9) aufweisen, im Bereich von 1 bis 3 mm liegt, während die in Bereichen zwischen einem jeweiligen Paar benachbarter Taillen-umgebender elastischer Elemente (9) gemessene (L3) im Bereich von 3 bis 10 mm liegt.

6. Artikel nach Anspruch 1, wobei die Taillen-umgebenden elastischen Elemente (9) im wesentlichen parallel zur Umfangskante (5a) der Taillenöffnung (5) verlaufen und ein jeweiliges Paar benachbarter Taillen-umgebender elastischer Elemente (9) in Längsrichtung einen Abstand im Bereich von 3 bis 30 mm voneinander aufweist.

7. Artikel nach Anspruch 1, wobei das Grundelement aus einer der Haut eines Trägers zugewandten flüssigkeitsdurchlässigen oberen Lage (18) und einer von der Haut des Trägers abgewandten flüssigkeitsundurchlässigen rückwärtigen Lage (19) gebildet ist, das flüssigkeitsabsorbierende Element (20) zwischen der oberen (18) und der rückwärtigen Lage (19) angeordnet ist und die Falten (10) in einem Bereich des Grundelements, der nicht vom flüssigkeitsabsorbierenden Element (20) eingenommen wird, praktisch kontinuierlich in der genannten Längsrichtung (Y), d.h. im wesentlichen senkrecht zu den Taillen-umgebenden elastischen Elementen (9) verlaufen und in der um die Taille umlaufenden Richtung (X) im wesentlichen mit gleichförmigen Abständen angeordnet sind.

## Revendications

1. Article vestimentaire jetable du type culotte, comprenant un élément structurel (2), constituant des régions de ceinture avant et arrière (M1, M3) opposées l'une à l'autre et une région d'entrejambes (M2) placée entre ces régions de ceinture, un élément d'absorption de liquides (12) s'étendant en travers de ladite région d'entrejambes (M2) jusque dans les régions de ceinture avant et arrière (M1, M3), un orifice de ceinture (5), et une paire d'orifices de jambe (6) au-dessous dudit orifice de ceinture (5), une pluralité d'éléments élastiques de ceinture (9), qui s'étendent dans une direction de ceinture (X), étant fixés dans un état étiré audit élément structurel (2) dans au moins l'une desdites régions de ceinture avant et arrière (M1, M3), et ledit élément structurel (2) étant doté d'une pluralité de fronces (10) générées par rétraction desdits éléments élastiques de ceinture (9), **caractérisé en ce que**
lesdits éléments élastiques de ceinture (9) sont disposés entre un bord périphérique (5a) dudit orifice de ceinture (5) et la région d'entrejambes (M2), en étant espacés l'un de l'autre d'une distance donnée dans une direction longitudinale dudit article sensiblement parallèlement audit bord périphérique (5a), lesdites fronces (10) étant formées à intervalles réguliers par des motifs répétés de creux (10b) et de crêtes (10a) dudit élément structurel (2) dans ladite direction de ceinture (X) s'étendant dans ladite direction longitudinale (Y) depuis ledit orifice de ceinture (5) vers ladite région d'entrejambes (M2) et perpendiculairement auxdits éléments élastiques de ceinture (9), des espaces (17) formés entre des crêtes (10a) et le torse d'un porteur étant ménagés de façon continue dans la direction longitudinale (Y) depuis la région d'entrejambes (M2) vers l'orifice de ceinture (5) suivant des lignes généralement droites, et le rétrécissement, dans la direction de ceinture(X), dudit élément structurel (2) dans lesdites régions (M1, M3) comportant lesdits éléments élastiques de ceinture (9), étant dans une gamme de 35 à 80% par unité de longueur.

2. Article selon la revendication 1, dans lequel ledit élément structurel (2) présente des creux (10b) et des crêtes (10a) agencés alternativement et générés par lesdits éléments élastiques de ceinture (9), et la hauteur maximale (L2) desdites fronces (10) entre des creux respectifs (10b) et des crêtes respectives (10a), mesurée dans des régions comportant chacune ledit élément élastique de ceinture (9), est inférieure à la hauteur (L3) mesurée dans des régions entre chaque paire d'éléments élastiques de ceinture adjacents (9).

3. Article selon la revendication 1, dans lequel les crêtes (10a) et les creux (10b) desdites fronces (10) ont tous sensiblement une forme d'arc de cercle s'étendant dans ladite direction de ceinture (X).

4. Article selon la revendication 1, dans lequel la distance entre les crêtes (10a) desdites fronces adjacentes (10) dans ladite direction de ceinture (X) est de l'ordre de 3 à 30 mm.

5. Article selon la revendication 2, dans lequel la hauteur maximale (L2) desdites fronces (10) entre les creux respectifs (10b) et les crêtes respectives (10a), mesurée dans lesdites régions comportant chacune ledit élément élastique de ceinture (9), est de l'ordre de 1 à 3 mm, tandis que la hauteur (L3) mesurée dans des régions entre chaque paire d'éléments élastiques de ceinture adjacents (9) est de l'ordre de 3 à 10 mm.

6. Article selon la revendication 1, dans lequel lesdits éléments élastiques de ceinture (9) s'étendent sensiblement parallèlement audit bord périphérique (5a) dudit orifice de ceinture (5), et chaque paire desdits éléments élastiques de ceinture adjacents (9) dans ladite direction longitudinale est espacée l'une de l'autre d'une distance de l'ordre de 3 à 30 mm.

7. Article selon la revendication 1, dans lequel ledit élément structurel est formé d'une feuille supérieure (18) perméable aux liquides, dirigée vers la peau du porteur, et d'une feuille arrière (19) imperméable aux liquides, dirigée du côté opposé à la peau du porteur, ledit élément d'absorption de liquides (20) étant interposé entre ladite feuille supérieure (18) et ladite feuille arrière (19), et lesdites fronces (10) s'étendant, dans une région dudit élément structurel non occupée par ledit élément d'absorption de liquides (20), pratiquement en continuité dans ladite direction longitudinale (Y), c'est-à-dire essentiellement perpendiculairement auxdits éléments élastiques de ceinture (9), et étant disposés essentiellement à intervalles réguliers dans ladite direction de ceinture (X).
